# EUROPEAN PATENT APPLICATION

(11) **EP 4 728 867 A1**
(43) Date of publication of application: **22.04.2026**
(21) Application number: 25206947.1
(22) Date of filing: 06.10.2025
(51) Int. Cl.: A01N 63/40, A61K 35/76, C12N 7/00

(54) **BACTERIOPHAGE COMPOSITION AGAINST BACTERIAL CANKER IN SOLANACEAE**

(30) Priority: 17.10.2024 BE 202405698
(71) Applicant: De Ceuster Meststoffen NV, 2280 Grobbendonk (BE)
(72) Inventor: HANSSEN, Inge Renée Maria, 2280 Grobbendonk (BE); VOS, Christine, 2280 Grobbendonk (BE); LUYPAERT, Gil Karel, 2280 Grobbendonk (BE)
(74) Representative: Arnold & Siedsma

(57) **Abstract**

The present invention relates to a bacteriophage composition for the treatment a plant of the *Solanaceae* family to protect the plants from infection caused by *Clavibacter michiganensis,* and more specifically *Clavibacter michiganensis* subsp. *michiganensis,* the causal agent of the disease called bacterial canker. The present invention further relates to methods for the treatment of a plant of the *Solanaceae* family to control *Clavibacter michiganensis* infection using said composition and use of the bacteriophage composition or bacteriophage for the treatment of a plant of the *Solanaceae* family against *Clavibacter michiganensis.*

## Description

The present invention relates to a bacteriophage composition for the treatment of a plant of the *Solanaceae* family to protect the plants from infection caused by *Clavibacter michiganensis.,* and more specifically *Clavibacter michiganensis* subsp. *michiganensis,* the causal agent of the disease called bacterial canker. The present invention further relates to methods for the treatment of a plant of the *Solanaceae* family to control *Clavibacter michiganensis* infection using said composition and use of the bacteriophage composition or bacteriophage for the treatment of a plant of the *Solanaceae* family against *Clavibacter michiganensis.*

Bacterial canker in *Solanaceae* refers to a disease caused by bacteria that affects plants in the *Solanaceae* family. The Solanaceae family includes important crops such as tomatoes, peppers, eggplants, and potatoes. One of the key bacterial pathogens associated with bacterial canker in *Solanaceae* is the Gram-positive bacterium *Clavibacter michiganensis* subsp. *michiganensis (Cmm),* first identified in 1909 in Michigan USA. This vascular pathogen generally invades and proliferates in the xylem through natural openings or wounds, causing wilt and canker symptoms. Symptoms of bacterial canker in *Solanaceae* may include wilting, necrosis, yellowing of leaves, cankers on stems, and the production of bacterial ooze from infected areas, resulting in reduced yields and reduced marketability of fruits showing lesions. Internal bacterial pathogens clog plant vessels resulting in brown stems. The bacteria typically enter the plant through wounds, natural openings, or during transplanting. Infected seeds can also serve as a source of the pathogen. Infected plants eventually desiccate and die.

Bacterial canker caused by *Cmm* is now widespread in Africa, Asia, Europe, North- and South America and Oceania. The most important crop affected by *Cmm* is tomato, where yield losses are variable but can be very severe, estimated in Ontario, Canada up to 84% in commercial fields, 46 to 93% plant death and approximately 50% decrease in average fruit weight (Sen *et al.* 2015, Peritore *et al.* 2021).

Control efforts include plants sprayed with chemical bactericides including copper compounds (copper sulphide, copper (hydr)oxide, and copper oxychloride), but such control strategies are not always effective, and are not sustainable, nor safe for consumers and workers and not environmentally friendly. Although these antimicrobial compounds can sometimes result in efficient reduction of bacterial titres, they are ultimately inadequate to protect the plant, and some are phytotoxic or promote resistance. Furthermore, the use of copper-based products is also being restricted more and more due to increasing environmental concerns and legislation.

There are little to no sustainable and effective plant protection products against bacterial canker infection allowed to be used in the EU. Governments decided to implement integrated pest management strategies (IPMs) as a standard for crop protection (e.g. Directive 2009/128/EC). In an attempt to prevent new *Cmm* infections, hygiene protocols have been developed that include measures to prevent the occurrence and spread of mechanically transmitted pathogens, including preparation of greenhouses, applying crop rotation to reduce a (potential) infection, and planning of planting and cultivation periods. However, such efforts are often suboptimal and require a lot of effort. Other control options include selecting for resistant cultivars, however most commercially successful cultivars at present still lack effective bacterial canker resistance. At present, when bacterial canker infections are observed, the only effective treatment is to remove and dispose of all plants from the infected area to avoid further spread of the disease.

Considering the above, there is a need in the art for an effective, safe and sustainable treatment against bacterial canker infection in plants, more specifically against *Cmm* infection. In addition, there is a need in the art for a method for an effective and sustainable treatment of plants that are affected by bacterial canker, more specifically *Cmm* infection. The treatment should provide a greener alternative and preferably not be affected by the increasing limiting environmental legislation.

It is an object of the present invention, amongst other objects, to address the above need in the art. The object of present invention, amongst other objects, is met by the present invention as outlined in the appended claims.

Specifically, the above object, amongst other objects, is met, according to a first aspect, by the present invention by a bacteriophage composition for the treatment of a plant of the *Solanaceae* family against *Clavibacter michiganensis* infection, wherein the composition comprises a bacteriophage CLAVIPHAGE7 having a genome sequence having at least 90% sequence identity, preferably at least 91%, 92%, 93%, 94%, 95%, 96%, 97%, 98%, 99% sequence identity, more preferably at least 99,5% sequence identity, even more preferably at least 99,9%, most preferably 100% sequence identity with SEQ ID No. 1, and the composition further comprises a bacteriophage CLAVIPHAGE9 having a genome sequence having at least 90% sequence identity, preferably at least 91%, 92%, 93%, 94%, 95%, 96%, 97%, 98%, 99% sequence identity, more preferably at least 99,5% sequence identity, even more preferably at least 99,9%, most preferably 100% sequence identity with SEQ ID No. 2.

Bacteriophages are a group of viruses that rely on bacteria for survival and replication. Bacteriophages consist of genetic material (DNA or RNA, single- or double stranded, circular or linear; mostly dsDNA is found) surrounded by a protein capsid as is the case for the bacteriophages comprised in the composition of present invention. Bacteriophages are the most abundant biological entity in the biosphere with an estimated number of 10³¹ individuals, which is at least ten times higher than the number of bacterial cells. They can be found in saltwater, freshwater, soil, plants and animals as well as in the human digestive and genitourinary tracts and even on the skin. Bacteriophages are the natural enemies of bacteria and fit nicely within the scope of the implemented integrated pest management strategies (IPMs) as a standard for crop protection (e.g. Directive 2009/128/EC). Genome sequencing has revealed a huge diversity in bacteriophage genomes, at current over 19.000 genomes of viruses are included in the NCBI database, the majority of which being bacteriophages.

According to yet another preferred embodiment, the present invention relates to the bacteriophage composition, wherein the *Clavibacter michiganensis* infection is caused by the *Clavibacter michiganensis* subsp. *michiganensis* bacterium. For example, the *Clavibacter michiganensis* subsp. *michiganensis* represented by isolate DSM 34789 as deposited at Leibniz-Institut DSMZ-Deutsche Sammlung von Mikroorganismen und Zellkulturen GmbH, Inhoffenstr. 7B, D38124 Braunschweig, Germany on 4^{th} October 2023.

Bacteriophage host recognition occurs via receptors on the cell surface. Bacteriophages have a narrow host range and are generally considered species- or even strain-specific. The host specificity of bacteriophages can vary per genus. Some bacteriophages can infect members of multiple bacterial genera while some are specific for particular isolates or groups of isolates of closely related host species. In order to successfully infect a host bacterium, binding of the bacteriophage to the bacterium is needed with specialized surface receptors. As a consequence, it is unlikely that bacteriophages that are able to infect bacteria with specific receptors are also able to infect commensal bacteria. Experiments show that the bacteriophages of the composition of present invention are strictly specific to *Clavibacter michiganensis* , more specifically *Clavibacter michiganensis* subsp. *michiganensis.* Therefore, the bacteriophage composition of present invention does also not affect other bacteria that are beneficial to the plant or orchard on which the composition is applied for treatment of bacterial canker.

According to another preferred embodiment, the present invention relates to the bacteriophage composition, wherein the plant of the *Solanaceae* family is selected from the group consisting of *Solanum lycopersicum* (tomato), *Solanum tuberosum* (potato), *Capsicum annuum* (pepper), *Solanum melongena* (eggplant), *Capsicum frutescens, Solanum pectinatum, Solanum quitoense, Solanum aethiopicum, Solanum Americanum, Solanum atropurpureum, Solanum aviculare, Solanum capsicoides, Solanum carolinense, Solanum dulcamara, Solanum myriacanthum, Solanum pseudocapsicum, Solanum scabrum, Solanum violaceum,* preferably tomato, potato or pepper.

According to another preferred embodiment, the present invention relates to the bacteriophage composition, wherein bacteriophage CLAVIPHAGE7 is obtainable from the deposit DSM 34861, and/or wherein bacteriophage CLAVIPHAGE9 is obtainable from the deposit DSM 34862. The above specified bacteriophages were deposited at Leibniz-Institut DSMZ-Deutsche Sammlung von Mikroorganismen und Zellkulturen GmbH, Inhoffenstr. 7B, D38124 Braunschweig, Germany on 4^{th} October 2023.

According to another preferred embodiment, the present invention relates to the bacteriophage composition, wherein the composition is a suspension concentrate, an aqueous solution, granule or powder, preferably suspension concentrate.

The composition of present invention may further comprise additives or adjuvants for improved efficacy, such as UV protectors for improved stability of the bacteriophage composition under sunlight, spreaders for increased contact surface and better uptake, or rain-fastness enhancers for improved persistence in rainy weather. Inclusion of spreaders in the composition of present invention provides for an improved spread of the bacteriophages when applied due to a larger contact surface, resulting in improved uptake and improved treatment of bacterial canker. Rain-fastness enhancers for example prevent washout of the bacteriophages during rainfall, preventing a reduced efficacy and maintaining high MOIs (Multiplicity of Infection). The MOI or multiplicity of infection refers to the ratio of bacteriophages to target bacterial cells and is an important value in bacteriophage-based control of bacterial diseases. The composition of present invention may further include sticker, emulsifier, wetting agent, buffering agent, anti-foam agent, drift control agent, additive for increased surface contact, reduced runoff, and increased leaf penetration in various weather conditions.

According to yet another preferred embodiment, the present invention relates to the bacteriophage composition, wherein the composition comprises 1x10⁷ to 1x10¹² PFU/mL of bacteriophages, preferably 1x10⁸ to 1x10¹¹ PFU/mL, more preferably 1x10⁹ to 1x10¹⁰ PFU/mL. Experiments show that a concentration of 1x10⁹⁻¹⁰ PFU/mL is especially suitable against the *Clavibacter michiganensis* infection, preferably *Clavibacter michiganensis* infection in tomato plants.

The present invention, according to a second aspect, relates to a method for protecting or treating a plant of the *Solanaceae* family against *Clavibacter michiganensis* infection by contacting said plant with a bacteriophage composition.

According to a preferred embodiment, the present invention relates to the method wherein the bacteriophage composition is applied to the plant, fruit, leave, blossom, shoot, root, or other plant parts. The bacteriophage composition of present invention can be used as preventive treatment to prevent the plant from being infected by *Clavibacter michiganensis* and affected by bacterial canker. Furthermore, and as shown in the experiments, when infected by *Clavibacter michiganensis,* tomato plants can be effectively treated by application of the composition of present invention to said plants.

According to a preferred embodiment, the present invention relates to the method wherein the method is a preventive and/or curative method.

According to another preferred embodiment, the present invention relates to the method wherein the application of the bacteriophage composition to the plant is performed by spraying, watering of the plant, irrigation, soil/substrate drenching, via a drip irrigation system on the soil/substrate or solid application via granules, dusting, dipping, via insect transmission, preferably by spraying.

According to another preferred embodiment, the present invention relates to the method, wherein the bacteriophage composition is applied at a dosage in the range of 0,375 to 7 L/ha LWA, preferably 0,875 to 4,5 L/ha LWA, more preferably 1,5 to 3,5 L/ha LWA. Preferably the bacteriophage composition is applied at a dosage of at least 0,875 L/ha LWA, preferably at least 1,5L/ha LWA, more preferably at least 3,5 L/ha LWA. The combination of concentration of the product (number of viable bacteriophages per ml of product, see above when discussing the MOI), and the dosage applied on the plant (in L per ground surface or ha leaf wall area (LWA)) of the product may be important to obtain an optimal efficacy. L/ha LWA (or 10000 m² LWA) is a dose expression for plant protection products referring to an amount (i.e. L) per treated hectare (ha) leaf wall area (LWA) of the plant, as further described in (Bulletin OEPP/EPPO Bulletin (2012) 42 (3), 409-41). Experiments show that when the bacteriophage composition is applied to the leaves of a plant suffering from bacterial canker the application at a dose of at least 0,875 L/ha LWA is already sufficient for reducing the *Cmm* infection pressure. As indicated in the experiments, preferably a dosage in the range of 1,5 to 3,5 L/ha LWA, more preferably 1,75 to 7 L/ha LWA is used for the treatment of bacterial canker in tomato.

According to another preferred embodiment, the present invention relates to the method, wherein the bacteriophage composition is applied to the plant at an application frequency selected from the group consisting of at least once a year, at least once a month, at least twice a month, at least four times a month, at least every two weeks, at least once a week, and at least twice a week, at least three times a week, and at least daily, preferably at least once or twice a week.

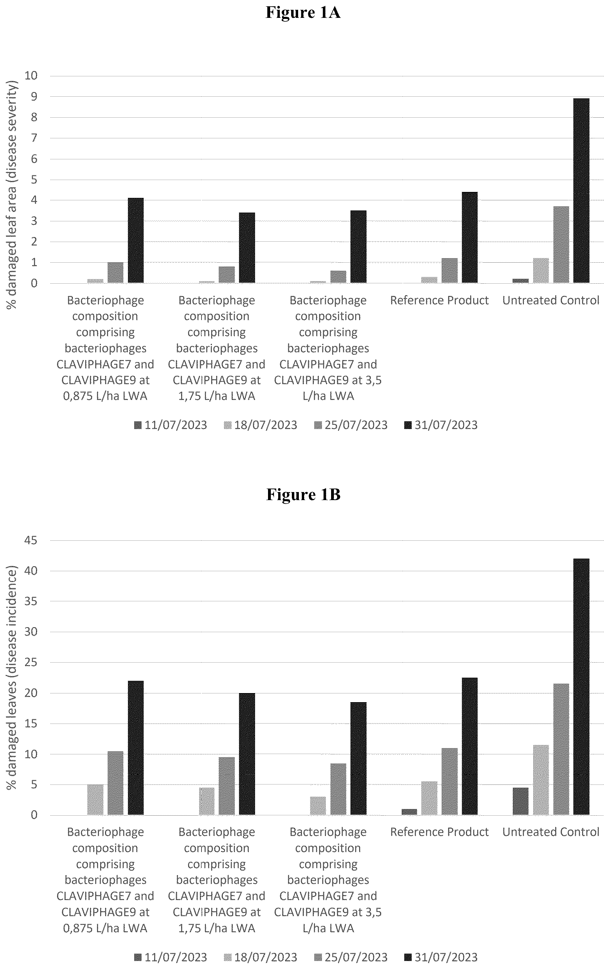

The method of present invention can prevent or reduce bacterial canker in infected plants by killing the *Clavibacter michiganensis,* wherein bacterial infection or bacterial canker incidence is reduced by at least 30%, preferably at least 40%, more preferably at least 45%, most preferably at least 50% in comparison to untreated plants. Reduction of bacterial infection or bacterial canker incidence can be determined by the skilled in the art for example by counting infected leaves or measurements of bacterial infection and determination of the reduction. For example, the percentage of affected leaf area, reflecting disease severity can be compared of plants that received treatment or the untreated plants. Assessments on the percentages of affected leaf area (reflecting disease severity, Figure 1A) and the percentage of affected leaves (reflecting disease incidence, Figure 1B) during the trial period recorded by visual inspection, of the *Cmm* infection and resulting bacterial canker in the tomato plants. Furthermore, assessments on the bactericidal efficacy of the composition comprising the CLAVIPHAGE phages and the reference product were calculated by Abbott's formula as described in Abbott, W. S. (1925). "A Method of Computing the Effectiveness of an Insecticide". Journal of Economic Entomology. 18 (2): 265-267, during the trial period.

The present invention, according to a further aspect, relates to the use of a bacteriophage composition or bacteriophage as described or claimed herein for the treatment prevention and/or control of a plant of the *Solanaceae* family affected by *Cmm* infection and/or bacterial canker. Isolated bacteriophage CLAVIPHAGE7 (SEQ ID No. 1) and/or CLAVIPHAGE9 (SEQ ID No. 2) can be used alone or in combination for the treatment of a plant of the *Solanaceae* family affected by bacterial canker infection. Combining two or more bacteriophages in the composition of present invention improves the antimicrobial effect, reduces bacterial escape, and improves treatment options.

The present invention, according to a further aspect, relates to an isolated bacteriophage species for the treatment of a plant of the *Solanaceae* family against *Clavibacter michiganensis,* comprised of a genome sequence having at least 95%, preferably at least 99%, most preferably at least 99,5% sequence identity with SEQ ID No. 1 or SEQ ID No. 2. A phage is considered to belong to the same species if the identity at the genome level is more than 95% and if there is a clear genome synteny.

The present invention, according to a further aspect, relates to an isolated bacteriophage for the treatment of a plant of the *Solanaceae* family against *Clavibacter michiganensis,* comprised of a genome sequence having at least 99,5% sequence identity, preferably at least 99,8% sequence identity, preferably 100% sequence identity with SEQ ID No. 1 or SEQ ID No. 2.

The present invention will be further detailed in the following examples and figures wherein:
- **Figure 1:**: Shows the disease incidence and disease severity of tomatoes infected by *Clavibacter michiganensis* subsp. *michiganensis* and suffering from bacterial canker. **Figure 1A** shows the percentage of affected leaf area, reflecting disease severity, during the trial period of tomato plants that received treatment or the untreated plants. **Figure 1B** shows the percentage of affected leaves, reflecting disease incidence, during the trial period of tomato plants that received treatment or the untreated plants. Plants that have been treated by the bacteriophage composition of present invention comprised of bacteriophages CLAVIPHAGE7 and CLAVIPHAGE9 at the different dosage rates (3,5; 1,75 and 0,875 L/ha LWA) and the reference product (KING 360 HP) showed that the bacterial canker could be effectively controlled by the bacteriophage composition of present invention comparable to the reference product.

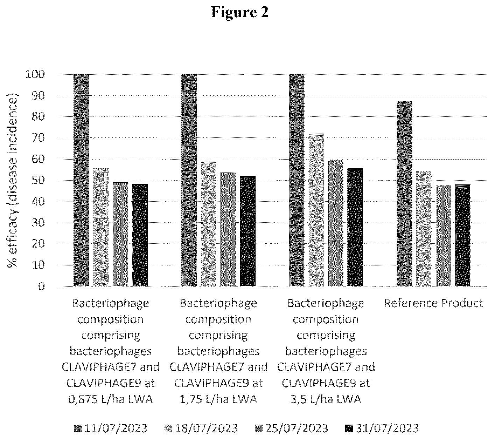

- **Figure 2:**: Shows the bactericidal efficacy of the composition comprising the bacteriophages CLAVIPHAGE7 and CLAVIPHAGE9 and the reference product (KING 360 HP), calculated by Abbott's formula during the trial period. The bacterial efficacy of the composition of present invention comprising the bacteriophages CLAVIPHAGE7 and CLAVIPHAGE9 is comparable or even slightly more effective against *Cmm* in comparison to the reference product.

### Examples

### Example 1 - sequencing of CLAVIPHAGE7 and CLAVIPHAGE9 bacteriophage

The full genomes of the *Clavibacter michiganensis* subsp. *michiganensis* bacteriophages comprised in the composition of present invention were sequenced using Illumina sequencing, more specifically CLAVIPHAGE7 and CLAVIPHAGE9. (SEQ ID No.1 and SEQ ID No. 2, respectively). From each bacteriophage total bacteriophage genomic DNA was extracted as previously described in (Kot, W. Genome sequencing of dsDNA-containing bacteriophages directly from a single plaque. Methods Mol Biol. 1681, 179-184 (2018)). Briefly, a 90 µL aliquot of the provided bacteriophage stock was mixed with 10 µL Dnase I buffer (ThermoFisher) and filtered using a 0,45 µm ultrafiltration spin-column (Merck Millipore) to remove any possibly remaining cell debris. Subsequently, the mixture was treated with 5 U Dnase I (30', 37°C) (ThermoFisher) to get rid of all external host DNA. After addition of 10 µL 50 mM EDTA and 10 µL 1% SDS, the bacteriophage capsids were digested with 3 U proteinase K (45', 55°C) (ThermoFisher). Finally, the total bacteriophage genomic DNA was purified using the DNA Clean & Concentrator-5 kit (Zymo Research). The eluted bacteriophage DNA was directly used for Illumina sequencing (Illumina MiniSeq platform). Using MEGA X v10.1.89, bacteriophage genomes were then aligned to the closest type species as identified by BLASTn10 and/or Viptree v1.911.

To study the taxonomy of the viruses in more detail, the virus intergenomic distance between the most related phages (as identified by BLASTn and Viptree) and the CLAVIPHAGE phages was calculated. For taxonomic analysis, the International Committee for the Taxonomy of Viruses (https://talk.ictvonline.org/taxonomy/) guidelines were followed (Adriaenssens and Rodney Brister, 2017; Turner, Kropinski and Adriaenssens, 2021). According to the rules set by the Bacterial and Archaeal Viruses Subcommittee (BAVS) of the International Committee on the Taxonomy of Viruses (ICTV). for phage taxonomy, a phage is considered to belong to the same species if the identity at the genome level is more than 95% and if there is a clear genome synteny. A genus on the other hand is defined as a cohesive group of viruses sharing a high degree of nucleotide sequence similarity (more than 70% and a high genome synteny), which is distinct from viruses of other genera. Based on these parameters, the closest known phages from the public databases were identified for each of the *Cmm* phages using BLASTn. This analysis revealed that the *Cmm* phages do not share any significant nucleotide sequence similarity with any known sequence in the NCBI database.

Proteome analysis clusters the CLAVIPHAGE phages the closest together with some *Arthrobacter,* one *Rhodococcus,* several *Gordonia,* two *Rhodobacter* and many *Streptomyces* phages. Representatives of each branch include *Arthrobacter* phage Decurro (unpublished) and BlueFeather (unpublished), the *Rhodococcus* phage RRH1 (unpublished), *Gordonia* phage GRU3 (Dyson et al., 2015) and finally *Streptomyces* phages mu1/6 (Farkasovska et al., 2004) and Chymera (unpublished). While these phages contain an integrase, suggesting a temperate lifestyle, the CLAVIPHAGE phages do not contain this integrase gene. Based on this proteome analysis, the phages are belonging to an unclassified family. Furthermore, after annotation of the genomes, no indications were found that would suggest these bacteriophages have a temperate lifestyle. It was concluded that the identified bacteriophages are likely strictly lytic, based on their known encoded proteins (genbank files) and on their related bacteriophages (BLASTn and Viptree analysis).

According to taxonomy guidelines, phages showing more than 95% and 70% sequence identity are classified together in one species or genus, respectively. CLAVIPHAGE9 (SEQ ID No.2), belongs to a new species within a novel genus of an unclassified family within the *Caudoviricetes* class. CLAVIPHAGE7 (SEQ ID No.1) shared only 36,7% to 37,4% sequence identity with CLAVIPHAGE9 and therefore forms another new species within another new unclassified genus according to the International Committee for the Taxonomy of Viruses (ICTV) guidelines.

### Example 2 - Host specificity of CLA VIPHAGE7 and CLAVIPHAGE9 bacteriophages

The host specificity test is a test to confirm that the bacteriophages in the products are specific to the problematic bacteria that they were isolated to destroy, and not infective to other closely related organisms or bacterial flora within plants, soil, or humans. Bacteriophages CLAVIPHAGE7 and CLAVIPHAGE9 of example 1 were tested for their host specificity on different bacterial species including *Rathayibacter rathayi* (ATCC 13659), *Rathayibacter tritici* (ATCC 11403), *Leifsonia aquatica* (ATCC 14665), *Frigoribacterium faeni* (ATTC BAA-3), *Leucobacter chromiireducens* (ATCC BAA-1336), *Subtercola boreus* (ATCC BAA-168), *Agrococcus citreus* (ATCC 700859), *Curtobacterium albidum* (ATCC 15831). The test substances are bacteriophage concentrates. For storage, they are refrigerated at 4°C.

Each bacteriophage was tested against eight organisms and observed for formation of lysis. A simple spotting assay is used, which is a quick method to determine lysis. Briefly, bacteria were inoculated on LBA plates and incubated overnight at 30°C. Next, 10 µL of each bacteriophage was spotted on the plate comprising the bacteria and subsequently incubated for 18 to 22 hours at 30°C. Next, plates were examined by eye for lysis or plaques of the bacteria for each bacteriophage spot. The lack of lysis or plaques indicates that the bacteriophages are not active against the tested host.

No cell lysis or plaques were observed when the bacteria were exposed to the CLAVIPHAGE7 and CLAVIPHAGE9 bacteriophages, whereas *Clavibacter michiganensis* subsp. *michiganensis* bacteria (e.g. the *Clavibacter michiganensis* subsp. *michiganensis* represented by deposit DSM 34789 as deposited at Leibniz-Institut DSMZ-Deutsche Sammlung von Mikroorganismen und Zellkulturen GmbH, Inhoffenstr. 7B, D38124 Braunschweig, Germany on 4 October 2023) did show cell lysis and plaques when exposed to the bacteriophages. It can be concluded that the selected bacteriophages are host specific for the *Clavibacter michiganensis* subsp. *michiganensis* bacteria.

### Example 3 - Host range of CLAVIPHAGE7 and CLAVIPHAGE9 bacteriophages on Clavibacter michiganensis subsp. michiganensis isolates

The host range of the CLAVIPHAGE7 and CLAVIPHAGE9 bacteriophages on *Clavibacter michiganensis* subsp. *michiganensis (Cmm)* isolates was investigated on 42 different isolates of *Clavibacter michiganensis* subsp. *michiganensis,* that originated from EU, Switzerland and Morocco, of which isolate 34 to 42 were collected most recently during the 2023 growing season (available at De Ceuster Meststoffen NV, Bannerlaan 79, Grobbendonk, Belgium). This is a relative test to measure the activity of the bacteriophages against the *Cmm* bacterial isolates.

The susceptibility of the *Cmm* isolates against the bacteriophages was determined by the Spot test method (Amorim et al., 2009). Briefly, soft agar (dipeptone 5 g/L, yeast 2 g/L, MgSO₄7H₂O 2 g/L, K₂HPO₄ 1 g/L, glycerol 5 mL/L, agar 6 g/L, pH 6,5±0,1) containing 100 µL of a bacterial cell suspension at optical density (OD₆₀₀ₙₘ) 0,5 was poured over bottom agar plates (peptone 5 g/L, yeast 2 g/L, MgSO₄.7H₂O 2 g/L, KH₂PO₄ 0,25 g/L, glycerol 5 mL/L, NaOH 0,01 g/L, agar 6 g/L, pH 6,4±0,1). Following solidification of the top agar matrix, 3 drops of 5 µL of a phage solution at 1E+07 PFU/mL pipetted on the top agar (Daubie et al., 2022).

Results were visualized as zones of clearing (halo) in the bacterial lawn. Halo formations were scored after 48h of incubation at 27°C. Host specificity was scored on the basis of standardized spot scoring on a scale of 0 to 6, wherein
0 = No clearing of the spot. Bacterial lawn grows evenly in and around the spot,
1 = The bacterial lawn may be affected by the phage. This may look similar to a watermark OR the spot contains <10 plaques,
2 = The spot is mostly a lawn with discrete plaques visible within it (≥10 plaques) OR is opaque (but clearly the phage is having an effect upon the lawn),
3 = The spot is cloudy,
4 = The spot background is faintly cloudy (hazy) or almost translucent,
5 = The spot is completely translucent, with some resistant bacterial colonies growing within the spot area, and
6 = The spot is completely translucent, with no bacteria growing within or on the spot area. Table 1. below provides a summary of the host range of the CLAVIPHAGE7 and CLAVIPHAGE9 bacteriophages on 42 different isolates of *Clavibacter michiganensis* subsp. *michiganensis,* on the basis of the above scoring.

**Table 1. Scoring of host range of the bacteriophages on isolates of Cmm**

| **Cmm Isolate** | **Country of origin** | **CLAVIPHAGE7** | **CLAVIPHAGE9** |
|---|---|---|---|
| 1 | Belgium | 4 | 2 |
| 2 | Belgium | 6 | 6 |
| 3 | Belgium | 5 | 4 |
| 4 | Belgium | 6 | 6 |
| 5 | France | 5 | 4 |
| 6 | Belgium | 6 | 6 |
| 7 | Switzerland | 3 | 0 |
| 8 | Belgium | 6 | 5 |
| 9 | Belgium | 5 | 5 |
| 10 | Belgium | 6 | 5 |
| 11 | Belgium | 6 | 5 |
| 12 | France | 6 | 6 |
| 13 | Morocco | 6 | 6 |
| 14 | Italy | 3 | 3 |
| 15 | Switzerland | 6 | 6 |
| 16 | Switzerland | 4 | 3 |
| 17 | Morocco | 6 | 6 |
| 18 | Morocco | 3 | 3 |
| 19 | Morocco | 3 | 3 |
| 20 | Morocco | 6 | 6 |
| 21 | Morocco | 6 | 5 |
| 22 | Morocco | 3 | 3 |
| 23 | Morocco | 4 | 4 |
| 24 | Morocco | 6 | 6 |
| 25 | Morocco | 6 | 4 |
| 26 | Morocco | 3 | 3 |
| 27 | France | 3 | 4 |
| 28 | Spain | 4 | 6 |
| 29 | Switzerland | 6 | 6 |
| 30 | Germany | 6 | 6 |
| 31 | Italy | 6 | 6 |
| 32 | Italy | 0 | 4 |
| 33 | Italy | 5 | 6 |
| 34 | France | 1 | 2 |
| 35 | France | 6 | 6 |
| 36 | France | 1 | 1 |
| 37 | France | 1 | 1 |
| 38 | Belgium | 6 | 6 |
| 39 | The Netherlands | 6 | 6 |
| 40 | France | 2 | 2 |
| 41 | France | 6 | 6 |
| 42 | France | 1 | 2 |

Isolates with scores of above 0 are considered as susceptible. The above results show that the selected bacteriophages are very effective against *Cmm.* As can be seen from Table 1, most *Cmm* isolates are susceptible to infection by both bacteriophages, although the level of activity can vary where specific isolates are controlled more efficiently than others, depending on the bacteriophage-bacterial isolate combination. Furthermore, these results support the idea that a combination (mixture) of two or more bacteriophages, can control the disease even more efficiently in view of bacterial diversity of the *Cmm* species. For *Cmm* isolate 7 only CLAVIPHAGE7 seems to show activity against this isolate, and vice versa for CLAVIPHAGE9 against isolate 32 for example. Using combinations of phages improves the antibacterial efficacy against *Cmm.*

### Example 4 - Efficacy of bacteriophage composition comprising bacteriophages CLAVIPHAGE7 and CLAVIPHAGE9 against Cmm infection in tomato

A trial with tomato plants (Maremagno variety) was set up in a greenhouse sited in Fondi, Latina district (Lazio region) - Southern Italy, in order to evaluate the efficacy and selectivity of a composition comprised of bacteriophages CLAVIPHAGE7 and CLAVIPHAGE9 of present invention against *Clavibacter michiganensis* subsp. *michiganensis (Cmm)* infection causing bacterial canker of tomatoes, in season 2023.

Three groups were treated with the bacteriophage composition (1x10⁹ PFU/mL) at different concentration by foliar spray, a second group did not receive the bacteriophage or any other treatment against *Cmm* (negative control) and a third group was treated by a reference antimicrobial product (KING 360 HP (authorisation number Italy: 12738), Diachem S.p.A., 360 g tribasic copper sulfate/L), according to manufacturer's instructions. The composition was applied at 0,875; 1,75 and 3,5 L/10000 m² LWA (or L/ha LWA) being treatments T1, T2 and T3, respectively. The composition was compared to the negative control and the reference product KING 360 HP applied at a rate of 200 mL/100 L being treatment T4. The untreated control group received only water. Ten applications were carried out during the trial period, starting from May 23rd to July 25th. The bacteriophage composition was applied during these months with a 7 day interval, just before sunset, using a backpack applicator, Oleo-Mac SP 126.

Assessments on the percentages of affected leaf area (reflecting disease severity, Figure 1A) and the percentage of affected leaves (reflecting disease incidence, Figure 1B) during the trial period were recorded by visual inspection, of the *Cmm* infection and resulting bacterial canker in the tomato plants. Furthermore, assessments on the bactericidal efficacy of the composition comprising the CLAVIPHAGE phages and the reference product were calculated by Abbott's formula as described in Abbott, W. S. (1925). "A Method of Computing the Effectiveness of an Insecticide". Journal of Economic Entomology. 18 (2): 265-267, during the trial period (Figure 2).

At the first efficacy assessment, which was carried out on 11th of July (the date of the 8th application), a percentage of 0,2% affected leaf area and 4,5% of affected leaves were observed in the untreated control, while no symptoms were recorded in the treated objects. On July 18th, the second efficacy assessment took place. At this date all the treatments showed disease symptoms with values of severity ranging from 0,1% to 1,2% without significant difference among the treated and the untreated objects. In terms of incidence all objects treated with the test item showed significantly lower values (3,0%-5,5%) compared to the untreated control (11,5%). The efficacy values in the treated objects, calculated by Abbott's formula, ranged from 73,4% to 82,3% on severity and from 55,6 to 71,9% on incidence. Although not significantly different from the value measured for the reference product, in absolute numbers the composition was at least comparable or even superior to the reference product KING 360 HP.

The third efficacy assessment was performed on July 25th. At this date, 3,7% of the leaf area was affected and 21,5 % of leaves were affected in T5 (untreated control), resulting in significantly higher values when compared to the test items with values of severity ranging from 0,6% (T3 - at 3,5 L/ha LWA) to 1,0% (T1 at 0,875 L/ha LWA) and incidence from 8,5% to 10,5% in T3 - at 3,5 L/ha LWA and T1 -at 0,875 L/ha LWA respectively. Efficacy values in the treated objects, calculated by Abbott's formula, ranged from 66,5% to 79,0% on severity and from 49,1% to 59,6% on incidence. Although not significantly different from the value measured in the reference treatment, in absolute numbers with the exception of the assessment on severity (T1 - at 0,875 L/ha LWA), they were all superior to the reference product KING 360 HP.

On July 31st, severity values between 3,4% and 4,1% were observed in the treated plots and a value of 4.4% was observed in the object treated with the reference product. These values were significantly lower than that observed in the untreated control (8.9%). Also in terms of incidence the untreated control (42,0%) resulted in statistically higher values compared to those obtained in the objects treated with the composition comprised of bacteriophages CLAVIPHAGE7 and CLAVIPHAGE9 (18,5%-22,0%) and the object treated with the reference product KING 360 HP (22,5%). The efficacy values in the treated objects, calculated by Abbott's formula, ranged from 51,8% to 55,2% on severity and from 48,3% to 55,8% on incidence. Again, although not significantly different from the value measured in reference treatment, in absolute numbers, with the exception of the assessment on severity (T1 - at 0,875 L/ha LWA), they were superior to the reference product KING 360 HP.

Results showed that, in the presence of a valid disease pressure of *Cmm,* the composition of present invention comprising the bacteriophages CLAVIPHAGE7 and CLAVIPHAGE9, and the reference product KING 360 HP showed significant disease control. A significant difference among the disease pressure (for both severity and incidence) between the untreated control and the treated were observed during the trial period. At 0,875; 1,75 and 3,5 L/10000 m² LWA the composition comprised of bacteriophages CLAVIPHAGE7 and CLAVIPHAGE9 showed significantly similar or even slightly improved performance to the reference product KING 360 HP. Even though no significant difference could be noticed between the different objects treated with the composition comprising the bacteriophages CLAVIPHAGE7 and CLAVIPHAGE9 and the object treated with the reference product, the efficacy of the reference product was inferior to that obtained at 1,75 or 3,5 L /10000 m² LWA with the composition. In half of the assessment dates the efficacy obtained after treatment with the reference product was slightly superior to that of the composition comprising the bacteriophages CLAVIPHAGE7 to CLAVIPHAGE9 at the lowest dose (0,875 L/10000 m² LWA). All treatments showed remarkable disease control during the entire trial period. No phytotoxic symptoms were observed on the plants in all the treatments where the composition comprised of bacteriophages and reference product KING 360 HP were applied. The bacteriophage composition of present invention provided a very efficient control of damages to the tomatoes, even applied at the lowest dose 0,875 L/ha LWA. These results show that the bacteriophage composition of present invention provides an effective treatment and/or control of bacterial canker in tomato.

A second experiment, identical to the experiment above was conducted early 2024 confirming the above results. Furthermore, a higher dosage of 7 L/ha LWA was also included which provided a comparable outcome in view of the 3,5 L/ha LWA dosage in view of disease control and efficacy of the composition comprising the bacteriophages CLAVIPHAGE7 and CLAVIPHAGE9.

## Claims

1. A bacteriophage composition for the treatment of a plant of the *Solanaceae* family against *Clavibacter michiganensis* infection, wherein the composition comprises a bacteriophage CLAVIPHAGE7 having a genome sequence having at least 90% sequence identity, preferably at least 98% sequence identity with SEQ ID No. 1, and the composition further comprises a bacteriophage CLAVIPHAGE9 having a genome sequence having at least 90% sequence identity, preferably at least 98% sequence identity with SEQ ID No. 2.

2. Bacteriophage composition according to claim 1, wherein the *Clavibacter michiganensis* infection is caused by the *Clavibacter michiganensis* subsp. *michiganensis* bacterium.

3. Bacteriophage composition according to claim 1 or 2, wherein the plant of the *Solanaceae* family is selected from the group consisting of *Solanum lycopersicum (tomato), Solanum tuberosum (potato), Capsicum annuum (pepper), Solanum melongena (eggplant), Capsicum frutescens, Solanum pectinatum, Solanum quitoense, Solanum aethiopicum, Solanum Americanum, Solanum atropurpureum, Solanum aviculare, Solanum capsicoides, Solanum carolinense, Solanum dulcamara, Solanum myriacanthum, Solanum pseudocapsicum, Solanum scabrum, Solanum violaceum,* preferably tomato, potato or pepper.

4. Bacteriophage composition according to any one of the claims 1 to 3, wherein the bacteriophage CLAVIPHAGE7 is obtainable from the deposit DSM 34861, and/or wherein the bacteriophage CLAVIPHAGE9 is obtainable from the deposit DSM 34862.

5. Bacteriophage composition according to any one of the claims 1 to 4, wherein the composition is a suspension concentrate, an aqueous solution, granule or powder, preferably suspension concentrate.

6. Bacteriophage composition according to any one of the claims 1 to 5, wherein the composition comprises 1x10⁷ to 1x10¹² PFU/mL of bacteriophages, preferably 1x10⁸ to 1x10¹¹ PFU/mL, more preferably 1x10⁹ to 1x10¹⁰ PFU/mL.

7. Bacteriophage composition according to any one of the claims 1 to 6, wherein the composition further comprises additives or adjuvants selected from the group consisting of a UV protector, spreader, rainfastness enhancer, sticker, emulsifier, wetting agent, buffering agent, anti-foam agent, drift control agent, additive for increased surface contact, reduced runoff and increased leaf penetration in various weather conditions.

8. A method for protecting or treating a plant of the *Solanaceae* family against *Clavibacter michiganensis* infection by contacting said plant with a bacteriophage composition according to any one of the claims 1 to 7.

9. The method according to claim 8, wherein the bacteriophage composition is applied to the plant, fruit, leave, blossom, shoot, root, or other plant parts.

10. The method according to claim 8 or 9, wherein the method is a preventive and/or curative method.

11. The method according to any one of the claims 8 to 10, wherein the application of the bacteriophage composition to the plant is performed by spraying, watering of the plant, irrigation, soil/substrate drenching, via a drip irrigation system on the soil/substrate or solid application via granules, dusting, dipping, via insect transmission, preferably by spraying, preferably wherein the bacteriophage composition applied at a dosage in the range of 0,375 to 7 L/ha LWA, preferably 0,875 to 4,5 L/ha LWA, more preferably 1,5 to 3,5 L/ha LWA..

12. The method according to any one of the claims 8 to 11, wherein the bacteriophage composition is applied to the plant at an application frequency selected from the group consisting of at least once a year, at least once a month, at least twice a month, at least four times a month, at least every two weeks, at least once a week, at least twice a week, at least three times a week, and at least daily, preferably at least once or twice a week.

13. The method according to any one of the claims 8 to 12, wherein the bacteriophage composition prevents or reduces bacterial canker in infected plants by killing the *Clavibacter michiganensis,* preferably wherein bacterial infection or bacterial canker incidence is reduced by at least 30%, preferably at least 40%, more preferably at least 45%, most preferably at least 50% in comparison to untreated plants.

14. Use of a bacteriophage composition of any one of the claims 1 to 7 for the treatment, prevention and/or control of *Clavibacter michiganensis* infection of a plant of the *Solanaceae* family.

15. An isolated bacteriophage species for the treatment of a plant of the *Solanaceae* family against *Clavibacter michiganensis,* comprised of a genome sequence having at least 95%, preferably at least 99% sequence identity with SEQ ID No. 1 or SEQ ID No. 2, preferably comprised of a genome sequence having at least 99.5% sequence identity, preferably at least 99,8% sequence identity, preferably 100% sequence identity with SEQ ID No. 1, more preferably comprised of a genome sequence having at least 99.5% sequence identity, preferably at least 99,8% sequence identity, preferably 100% sequence identity with SEQ ID No. 2.
